# EUROPEAN PATENT APPLICATION

(11) **EP 4 772 123 A1**
(43) Date of publication of application: **08.07.2026**
(21) Application number: 24859092.9
(22) Date of filing: 13.06.2024
(51) Int. Cl.: A61B 18/14, A61B 17/94

(54) **MEDICAL DEVICE AND ENDOSCOPIC TREATMENT INSTRUMENT**

(30) Priority: 31.08.2023 JP 2023140678
(71) Applicant: ASAHI INTECC CO., LTD., Seto-shi, Aichi, 489-0071 (JP)
(72) Inventor: TERAUCHI, Mikio, Seto-shi, Aichi 489-0071 (JP); WAKISAKA, Toshiaki, Seto-shi, Aichi 489-0071 (JP); TAKAHASHI, Hiroaki, Seto-shi, Aichi 489-0071 (JP)
(74) Representative: Winter, Brandl - Partnerschaft mbB
(86) International application number: PCT/JP2024/021451
(87) International publication number: WO 2025/047046

(57) **Abstract**

A medical device includes an endoscope and an endoscopic treatment instrument. An insertion portion of the endoscope includes a treatment instrument lumen and a curved part. The endoscopic treatment instrument includes a tube having a knife wire lumen, a knife wire housed in the knife wire lumen, and a support member. The knife wire includes a first wire part including an exposed part, and a second wire part. In a specific state in which the endoscopic treatment instrument is inserted into the treatment instrument lumen and a portion from a position of a distal end of the exposed part of the knife wire exposed from the tube to a proximal end side by a predetermined distance is projected from an opening of the insertion portion, a distal end of the second wire part is located on a more distal end side than a proximal end of the curved part.

## Description

### TECHNICAL FIELD

The technology disclosed in the present specification relates to a medical device and an endoscopic treatment instrument.

### BACKGROUND ART

Endoscopic sphincterotomy (EST) is known as a treatment for choledocholithiasis. A medical device including an endoscope and an incision instrument as an endoscopic treatment instrument is used for endoscopic sphincterotomy. An insertion portion of the endoscope is formed with a treatment instrument lumen that opens at a distal end portion of the insertion portion. The incision instrument is inserted into the treatment instrument lumen of the endoscope, and biological tissue is incised by a distal end portion of the incision instrument projected from an opening on a distal end side of the treatment instrument lumen.

An incision instrument used for endoscopic sphincterotomy includes a tube having a wire lumen, a knife wire (cutting wire) housed in the wire lumen, and a support member that rotatably supports a proximal end portion of the knife wire (see, for example, Patent Literature 1). A part of the knife wire (hereinafter, referred to as an "exposed part") is exposed to an outer peripheral side of the tube at all times or when the tube is in a predetermined posture. By applying a high-frequency current to the knife wire from a high-frequency power source, biological tissue can be incised by the exposed part of the knife wire.

An operator performs an operation of rotating the support member of the incision instrument in order to incise biological tissue in a predetermined range of a papilla into a predetermined shape. As a result, a rotational torque is generated at the proximal end portion of the knife wire supported by the support member, and the rotational torque is transmitted to the tube via the knife wire. As a result, the tube rotates around a central axis, and the direction of the exposed part of the knife wire changes to a desired direction.

### CITATION LIST

### Patent Literature

Patent Literature 1: Japanese Patent No. 4896351

### DISCLOSURE OF INVENTION

### TECHNICAL PROBLEM

The insertion portion of the endoscope has a curved part that is bent by a bending operation on a proximal end side of a distal end portion of the insertion portion where an opening of the treatment instrument lumen is formed. When the curved part of the endoscope is bent, the knife wire of the incision instrument inserted into the treatment instrument lumen of the endoscope is also bent. In this state, a rotational torque generated by rotating the support member that supports the proximal end of the knife wire is not favorably transmitted to the distal end side in a portion of the knife wire located in the curved part, and it may not be possible to smoothly change the direction of the exposed part of the knife wire to a desired direction. It is conceivable to reduce a diameter of a part of the tube to lower torsional rigidity of the part, but in that case, inner and outer diameters of the part become smaller, and it is not possible to form a lumen or the like of a necessary size. It is also conceivable to increase an outer diameter of the knife wire to improve rotational torque transmissibility, but increasing the outer diameter of the knife wire may reduce an incision capability of the knife wire.

Thus, in the related-art incision instrument, there is an issue that it is not possible to smoothly change the direction of the exposed part of the knife wire to a desired direction by a rotation operation of the support member while maintaining the inner and outer diameters of the tube and the outer diameter of the knife wire.

It is noted that such an issue is not limited to the endoscopic treatment instrument and the medical device used for endoscopic sphincterotomy for incising a papilla, but is a common issue for all endoscopic treatment instruments and medical devices that are inserted into various organs in a human body, such as a vascular system, a lymphatic system, a biliary system, a urinary system, a respiratory system, a digestive system, a secretory gland, and a reproductive organ, to incise biological tissue.

The present specification discloses a technology capable of addressing the above-described issue.

### SOLUTION TO PROBLEM

The technology disclosed in the present specification can be realized, for example, as the following aspects.
(1) A medical device disclosed in the present specification includes an endoscope having an insertion portion to be inserted into a subject, and an endoscopic treatment instrument. The insertion portion of the endoscope includes a treatment instrument lumen that opens at a distal end portion of the insertion portion and into which the endoscopic treatment instrument is inserted, and a curved part that is located on a more proximal end side than the distal end portion and is bent by a bending operation. The endoscopic treatment instrument includes a tube having a knife wire lumen, a knife wire housed in the knife wire lumen, and a support member that rotatably supports a proximal end portion of the knife wire. The knife wire includes a first wire part including an exposed part that can be exposed to an outer peripheral side of the tube, and a second wire part that is located on a more proximal end side than the first wire part and has higher rigidity than the exposed part of the first wire part. In a specific state in which the endoscopic treatment instrument is inserted into the treatment instrument lumen and a portion from a position of a distal end of the exposed part of the knife wire exposed from the tube to a proximal end side by a predetermined distance is projected from an opening of the insertion portion, a distal end of the second wire part is located on a more distal end side than a proximal end of the curved part.
   According to this medical device, in the specific state, the distal end of the second wire part having higher rigidity than the first wire part of the knife wire is located on a more distal end side than the proximal end of the curved part of the endoscope. That is, in the specific state, the portion of the knife wire having higher rigidity than the first wire part is located in the curved part of the endoscope. Therefore, even when the curved part of the endoscope is bent and the knife wire is bent, a rotational torque generated by rotating the support member is favorably transmitted to the distal end side even in the portion of the knife wire located in the curved part, and the direction of the exposed part of the knife wire can be smoothly changed to a desired direction. Therefore, according to this medical device, it is possible to smoothly change the direction of the exposed part of the knife wire to a desired direction by a rotation operation of the support member while maintaining the inner and outer diameters of the tube and the outer diameter of the knife wire.
(2) In the medical device described above, in the specific state, the distal end of the second wire part may be located on a more distal end side than a central position in a longitudinal direction of the curved part. According to this configuration, in the specific state, a portion of the knife wire having relatively high rigidity is located over a majority of the curved part of the endoscope in the longitudinal direction, so that a rotational torque generated by rotating the support member is more favorably transmitted to the distal end side, and the direction of the exposed part of the knife wire can be more smoothly changed to a desired direction.
(3) In the medical device described above, the first wire part may be cylindrical, and the second wire part may have a tapered shape that widens from a distal end side toward a proximal end side. According to this configuration, it is possible to provide the second wire part with relatively high rigidity while suppressing a rigidity gap in the knife wire.
(4) In the medical device described above, the knife wire may include a third wire part that is located on a more proximal end side than the second wire part, has higher rigidity than a distal end portion of the second wire part, and has a tapered shape that widens from a distal end side toward a proximal end side, and in the specific state, a distal end of the third wire part may be located on a more proximal end side than a proximal end of the curved part. According to this configuration, the third wire part having a relatively large diameter is not disposed in the curved part of the endoscope, and a decrease in slidability of the endoscopic treatment instrument in the treatment instrument lumen of the endoscope can be suppressed.
(5) In the medical device described above, the knife wire may include a third wire part that is located on a more proximal end side than the second wire part, has higher rigidity than a distal end portion of the second wire part, and is cylindrical, and in the specific state, a distal end of the third wire part may be located on a more proximal end side than a proximal end of the curved part. According to this configuration, the third wire part having a relatively large diameter is not disposed in the curved part of the endoscope, and a decrease in slidability of the endoscopic treatment instrument in the treatment instrument lumen of the endoscope can be suppressed.
(6) In the medical device described above, the knife wire may include a third wire part that is located on a more proximal end side than the second wire part, has higher rigidity than a distal end portion of the second wire part, and is cylindrical, and a fourth wire part that is located on a more proximal end side than the third wire part, has higher rigidity than a distal end portion of the third wire part, and has a tapered shape that widens from a distal end side toward a proximal end side, and in the specific state, a distal end of the third wire part may be located on a more distal end side than a proximal end of the curved part, and a distal end of the fourth wire part may be located on a more proximal end side than the proximal end of the curved part. According to this configuration, the fourth wire part having a relatively large diameter is not disposed in the curved part of the endoscope, and a decrease in slidability of the endoscopic treatment instrument in the treatment instrument lumen of the endoscope can be suppressed.
(7) In the medical device described above, a minimum value of a diameter of the exposed part of the knife wire may be 0.15 mm or more and 0.30 mm or less. According to this configuration, it is possible to achieve both an improvement in rotational torque transmissibility of the knife wire and an improvement in an incision capability of the knife wire.

The technology disclosed in the present specification can be realized in various forms, and for example, can be realized in the form of an incision instrument, an endoscopic treatment instrument, a medical device or a medical system including the incision instrument or the endoscopic treatment instrument and an endoscope, a method for manufacturing these devices and systems, and the like.

### BRIEF DESCRIPTION OF DRAWINGS

[FIG. 1] FIG. 1 is an explanatory diagram illustrating an external configuration of a medical device 300 according to a first embodiment.
[FIG. 2] FIG. 2 is an explanatory diagram illustrating an external configuration of the medical device 300 according to the first embodiment.
[FIG. 3] FIG. 3 is an explanatory diagram illustrating an external configuration of an incision instrument 100 according to the first embodiment.
[FIG. 4] FIG. 4 is an explanatory diagram illustrating a configuration of a cross section (XY cross section) of the incision instrument 100 at a position IV-IV in FIG. 3.
[FIG. 5] FIG. 5 is an explanatory diagram illustrating a configuration of a vertical cross section (YZ cross section) of a distal end portion of the incision instrument 100.
[FIG. 6] FIG. 6 is an explanatory diagram illustrating an example of an operation method of the incision instrument 100.
[FIG. 7] FIG. 7 is an explanatory diagram illustrating an example of an operation method of the incision instrument 100.
[FIG. 8] FIG. 8 is an explanatory diagram illustrating a detailed configuration of a knife wire 20 in the first embodiment.
[FIG. 9] FIG. 9 is an explanatory diagram illustrating a configuration of a knife wire 20X in a comparative example.
[FIG. 10] FIG. 10 is an explanatory diagram illustrating a detailed configuration of a knife wire 20a in a second embodiment.
[FIG. 11] FIG. 11 is an explanatory diagram illustrating a detailed configuration of a knife wire 20b in a third embodiment.

### EMBODIMENTS OF THE INVENTION

### A. First Embodiment:

### (Configuration of Medical device 300)

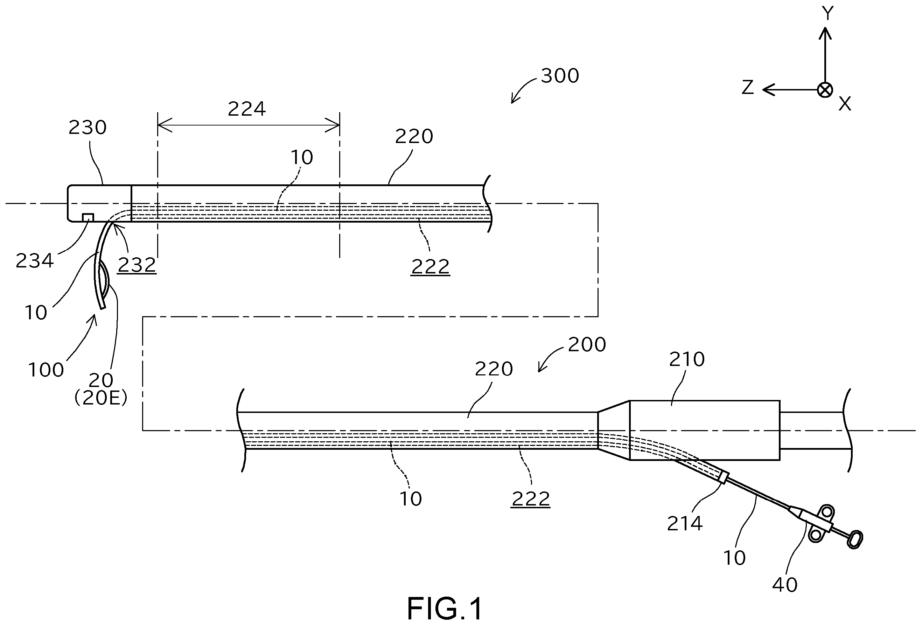

FIG. 1 is an explanatory diagram illustrating an external configuration of a medical device 300 according to a first embodiment. The medical device 300 of the present embodiment is used, for example, for endoscopic sphincterotomy (EST) for treatment of choledocholithiasis. It is noted that the medical device 300 is not limited to endoscopic sphincterotomy, and may be used for incising biological tissue by being inserted into various organs in a human body such as a vascular system, a lymphatic system, a biliary system, a urinary system, a respiratory system, a digestive system, a secretory gland, and a reproductive organ.

In FIG. 1, illustration of a part of the configuration of the medical device 300 is omitted as appropriate. FIG. 1 illustrates XYZ axes that are orthogonal to one another. A positive Z-axis direction side is a distal end side to be inserted into a body, and a negative Z-axis direction side is a proximal end side to be operated by an operator such as a physician. FIG. 1 illustrates a state in which a long portion of the medical device 300 is substantially linear and parallel to the Z-axis, but at least a part of the medical device 300 has flexibility to such an extent that it can be bent. These points are the same in the subsequent drawings. In the present specification, regarding the medical device 300 and its components, an end on the distal end side is referred to as a "distal end", the distal end and its vicinity are referred to as a "distal end portion", an end on the proximal end side is referred to as a "proximal end", and the proximal end and its vicinity are referred to as a "proximal end portion".

The medical device 300 includes an endoscope 200 and an incision instrument 100 as an endoscopic treatment instrument.

### (Configuration of Endoscope 200)

The endoscope 200 is an apparatus that is inserted into a subject (for example, in a lumen of a patient) to observe the inside. The endoscope 200 has an insertion portion 220 and a connection portion 210 connected to a proximal end of the insertion portion 220. The endoscope 200 is formed with a treatment instrument lumen 222, into which the incision instrument 100 is inserted, extending from the insertion portion 220 to the connection portion 210.

The insertion portion 220 of the endoscope 200 is a portion to be inserted into a subject, and is configured in a flexible and elongated shape. The insertion portion 220 has a distal end portion 230 and a curved part 224. On an outer peripheral surface of the distal end portion 230, an opening 232 on a distal end side of the treatment instrument lumen 222 is formed. A camera 234 is disposed on the outer peripheral surface of the distal end portion 230. An image signal generated by the camera 234 is transmitted to an image display device (not illustrated) connected to a proximal end portion of the endoscope 200 via a signal line (not illustrated), and an image is displayed on the image display device.

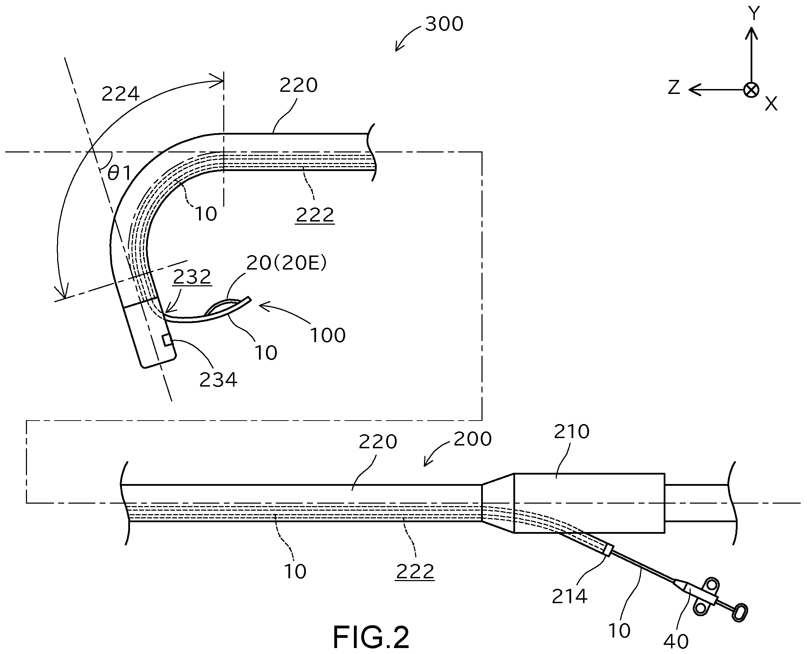

An operation wire (not illustrated) is connected to the curved part 224 of the insertion portion 220 of the endoscope 200. When an operation is performed on a bending operation portion (not illustrated) provided at the proximal end portion of the endoscope 200, tension is generated in the operation wire, and the curved part 224 is bent at a predetermined bending angle θ1. FIG. 2 illustrates a state in which the curved part 224 of the endoscope 200 is bent. A length of the curved part 224 along a longitudinal direction (Z-axis direction) is, for example, about 40 mm to 100 mm. A distance from the opening 232 to a distal end of the curved part 224 along the longitudinal direction is, for example, about 10 mm to 20 mm.

An insertion port 214 for an endoscopic treatment instrument is formed in the connection portion 210 of the endoscope 200. The insertion port 214 is an opening on a proximal end side of the treatment instrument lumen 222.

### (Configuration of Incision Instrument 100)

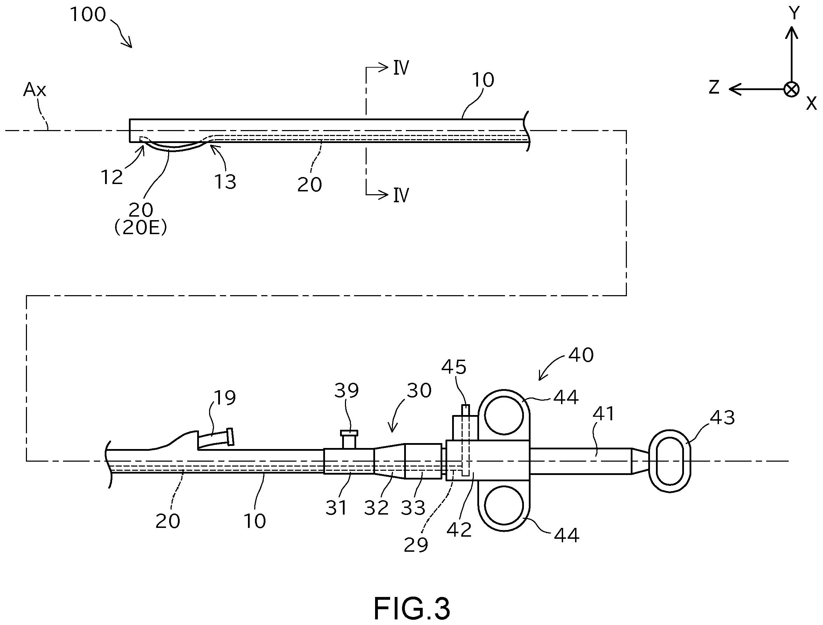

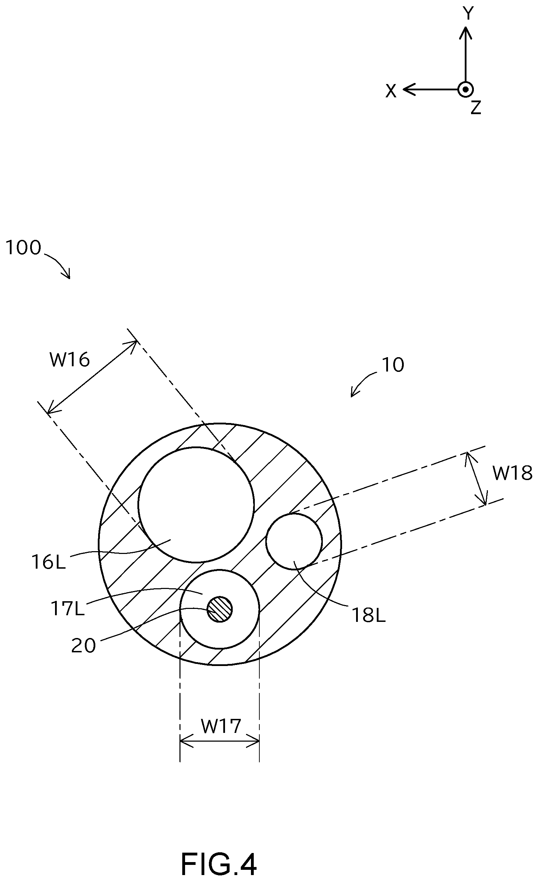

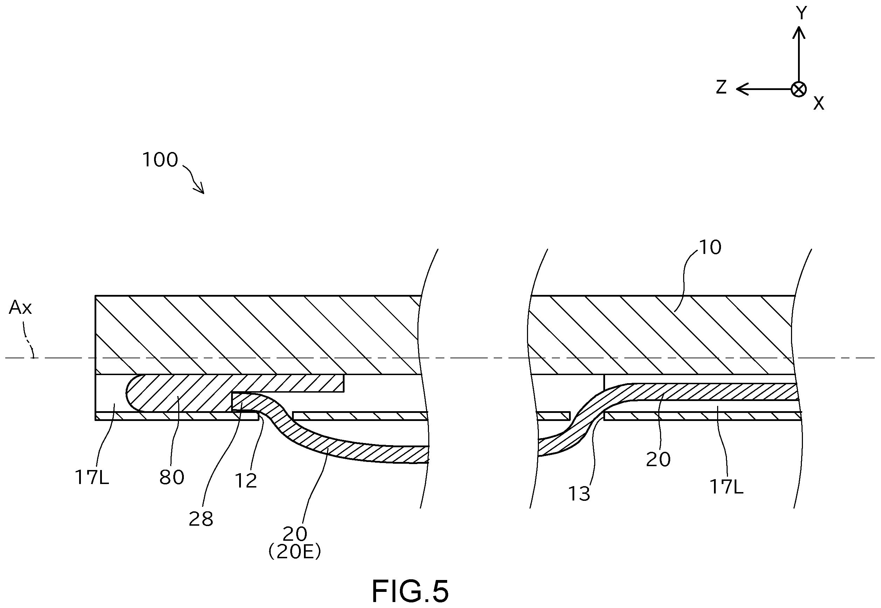

FIG. 3 is an explanatory diagram illustrating an external configuration of the incision instrument 100 according to the first embodiment, FIG. 4 is an explanatory diagram illustrating a configuration of a cross section (XY cross section) of the incision instrument 100 at a position IV-IV in FIG. 3, and FIG. 5 is an explanatory diagram illustrating a configuration of a vertical cross section (YZ cross section) of a distal end portion of the incision instrument 100. The incision instrument 100 is an instrument for incising biological tissue, and is used, for example, for endoscopic sphincterotomy for treatment of choledocholithiasis described above.

The incision instrument 100 includes a tube 10, a knife wire 20, a connector 30, and a handle portion 40. FIG. 3 illustrates a central axis Ax of the tube 10. In the present embodiment, central axes of the connector 30 and the handle portion 40 substantially coincide with the central axis Ax of the tube 10. However, these central axes may be different from the central axis Ax of the tube 10.

The tube 10 is an elongated member extending along the central axis Ax. As illustrated in FIG. 4, an outer shape of a cross section of the tube 10 is, for example, substantially circular or substantially elliptical. An outer diameter of the tube 10 may be constant over the entire length, or may change along the longitudinal direction.

The tube 10 is formed with a device lumen 16L, a knife wire lumen 17L, and a liquid feeding lumen 18L. The device lumen 16L is a lumen in which a combination device such as a guide wire is housed (inserted), and extends in the longitudinal direction from a distal end of the tube 10 to a branch portion 19 provided at a proximal end portion of the tube 10. The knife wire lumen 17L is a lumen in which the knife wire 20 is housed (inserted), and extends in the longitudinal direction from the distal end to a proximal end of the tube 10. The liquid feeding lumen 18L is a lumen for circulating a liquid such as a contrast agent or physiological saline, and extends in the longitudinal direction from the distal end to the proximal end of the tube 10. An outer shape of a cross section of each lumen is, for example, substantially circular or substantially elliptical. In the present embodiment, as illustrated in FIG. 4, a diameter W16 of the device lumen 16L is larger than a diameter W17 of the knife wire lumen 17L, and the diameter W17 of the knife wire lumen 17L is larger than a diameter W18 of the liquid feeding lumen 18L. A size relationship between the diameters of the respective lumens can be arbitrarily changed.

The tube 10 preferably has antithrombogenicity, flexibility, and biocompatibility, and can be formed of, for example, a resin material or a metal material. As the resin material for forming the tube 10, for example, a polyamide resin, a polyolefin resin, a polyester resin, a polyurethane resin, a silicone resin, a fluororesin, or the like can be used. As the metal material for forming the tube 10, for example, stainless steel such as SUS304, a Ni-Ti alloy, a Ni-Cr alloy, or the like can be used.

The knife wire 20 is an elongated member for incising biological tissue. The knife wire 20 is housed in the knife wire lumen 17L formed in the tube 10. A distal end portion of the knife wire 20 is fixed to a distal end portion of the tube 10. More specifically, as illustrated in FIG. 5, a fixing member 80 is filled in a distal end portion of the knife wire lumen 17L, and a distal end portion 28 of the knife wire 20 is fixed to the distal end portion of the tube 10 by being fixed to the fixing member 80. A length of a portion of the knife wire 20 embedded in the fixing member 80 is, for example, about 1 mm. As illustrated in FIG. 3, a proximal end portion 29 of the knife wire 20 extends from a proximal end portion of the knife wire lumen 17L to a position of the handle portion 40 via a lumen of the connector 30, and is supported by a wire support portion 45, which will be described later, of the handle portion 40.

A part of the knife wire 20 (hereinafter, referred to as an "exposed part 20E") is exposed to an outer peripheral side of the tube 10 between the distal end and the proximal end of the tube 10. More specifically, as illustrated in FIG. 5, the tube 10 is formed with a distal end side hole portion 12 and a proximal end side hole portion 13 that communicate the knife wire lumen 17L with the outside. In the knife wire 20, a portion on a proximal end side of the distal end portion 28 fixed to the fixing member 80 in the knife wire lumen 17L is exposed to the outside of the tube 10 via the distal end side hole portion 12, and a further proximal end side portion is housed again in the knife wire lumen 17L via the proximal end side hole portion 13. The exposed part 20E of the knife wire 20 is a portion from a position passing through the distal end side hole portion 12 to a position passing through the proximal end side hole portion 13. A length of the exposed part 20E of the knife wire 20 is, for example, about 20 mm to 30 mm. A minimum value of a diameter of the exposed part 20E of the knife wire 20 is, for example, 0.15 mm or more and 0.30 mm or less. The minimum value of the diameter of the exposed part 20E of the knife wire 20 may be, for example, 0.18 mm or more and 0.25 mm or less. In the present embodiment, the distal end side hole portion 12 and the proximal end side hole portion 13 are formed on an outer peripheral surface of the tube 10.

A high-frequency current is applied to the knife wire 20 from a high-frequency power source (not illustrated). The high-frequency power source is connected to the wire support portion 45. When the high-frequency power source is connected to the wire support portion 45, a conductive material exemplified by a metal can be used as a material for forming the wire support portion 45. As a result, biological tissue can be incised by the exposed part 20E of the knife wire 20.

The knife wire 20 preferably has conductivity, antithrombogenicity, and biocompatibility, and can be formed of, for example, a metal material, such as a stainless-steel alloy such as SUS302, SUS304, or SUS316, a Ni-Ti alloy, a Ni-Cr alloy, a cobalt alloy, gold, platinum, tungsten, or an alloy including these elements.

As illustrated in FIG. 3, the connector 30 is a tubular member connected to the proximal end portion of the tube 10. The connector 30 has a small-diameter portion 31, a tapered portion 32, and a large-diameter portion 33. The small-diameter portion 31 is a portion that constitutes a distal end portion of the connector 30 and has a substantially constant outer diameter. The large-diameter portion 33 is a portion that constitutes a proximal end portion of the connector 30 and has a substantially constant outer diameter larger than the outer diameter of the small-diameter portion 31. The tapered portion 32 is provided between the small-diameter portion 31 and the large-diameter portion 33, and is a portion in which an outer diameter gradually increases from a boundary with the small-diameter portion 31 to a boundary with the large-diameter portion 33.

A branch portion 39 extending in a direction intersecting the central axis Ax is formed in the small-diameter portion 31 of the connector 30. A lumen of the branch portion 39 communicates with the liquid feeding lumen 18L formed in the tube 10. Therefore, a liquid such as a contrast agent or physiological saline can be supplied to the liquid feeding lumen 18L via the branch portion 39.

The handle portion 40 is disposed on a proximal end side of the connector 30. The handle portion 40 has a shaft portion 41 and a handle body portion 42. The shaft portion 41 is a substantially cylindrical member and is connected to a proximal end portion of the connector 30. A lumen of the shaft portion 41 communicates with the lumen of the connector 30. A ring-shaped first finger rest portion 43 is provided at a proximal end of the shaft portion 41.

The handle body portion 42 is a substantially cylindrical member, and is attached to the shaft portion 41 so as to be slidable along the central axis Ax. The handle body portion 42 is provided with a wire support portion 45 that projects into the lumen of the shaft portion 41, and the proximal end portion 29 of the knife wire 20 is supported by (fixed to) the wire support portion 45. A pair of ring-shaped second finger rest portions 44 are provided on a side surface of the handle body portion 42. The handle body portion 42 is an example of a support member.

The connector 30 and the handle portion 40 can be formed of, for example, a resin material. As the resin material for forming the connector 30 and the handle portion 40, for example, polyurethane, polypropylene, rigid polyvinyl chloride, a polycarbonate resin, an acrylic resin, or the like can be used.

### (Operation Method of Incision Instrument 100)

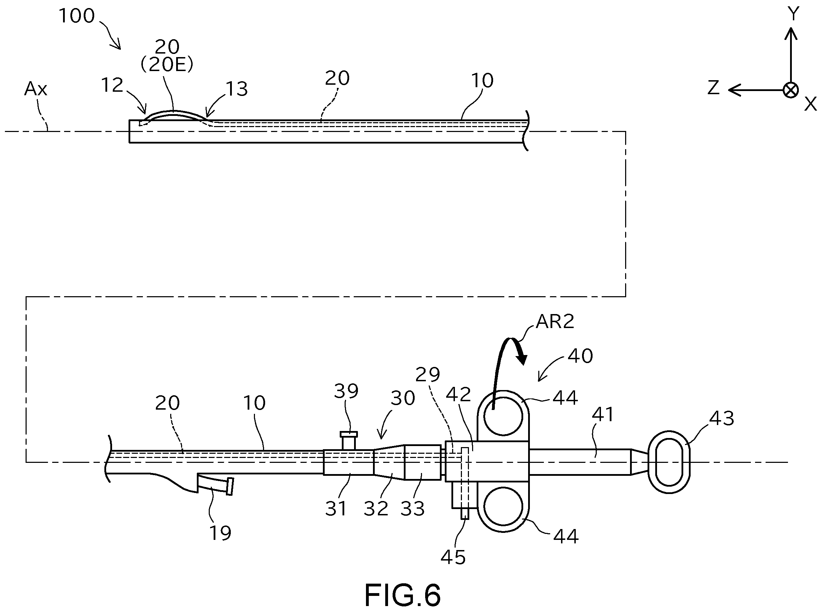

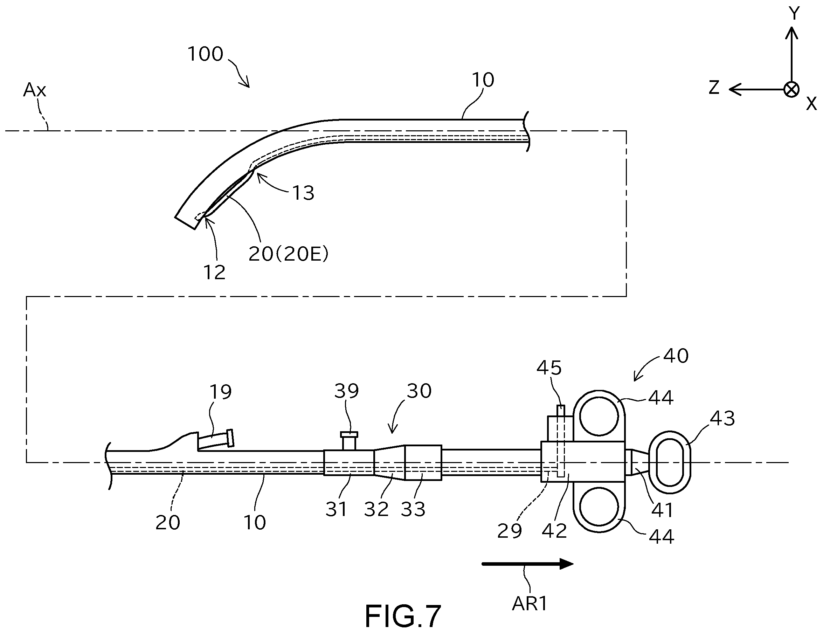

FIGS. 6 and 7 are explanatory diagrams illustrating an example of an operation method of the incision instrument 100. As illustrated in FIG. 6, an operator, for example, in a state in which a thumb is inserted into the first finger rest portion 43 and an index finger and a middle finger are inserted into the second finger rest portion 44, rotates the handle body portion 42 relative to the tube 10 (arrow AR2). Then, a rotational torque is generated at the proximal end portion 29 of the knife wire 20 supported by the wire support portion 45 of the handle body portion 42, and the rotational torque is transmitted to a distal end portion of the tube 10 fixed to a distal end portion of the knife wire 20 via the knife wire 20. As a result, the tube 10 rotates around the central axis Ax, and as a result, the direction of the exposed part 20E of the knife wire 20 changes. The incision instrument 100 may have a lock mechanism that fixes a rotation angle of the handle body portion 42 (that is, the direction of the exposed part 20E of the knife wire 20).

As illustrated in FIG. 7, when the operator, for example, in a state in which a thumb is inserted into the first finger rest portion 43 and an index finger and a middle finger are inserted into the second finger rest portion 44, pulls the index finger and the middle finger toward the proximal end side (arrow AR1), the handle body portion 42 moves to the proximal end side along an outer peripheral surface of the shaft portion 41. Then, tension is applied to the knife wire 20 by pulling the proximal end portion 29 of the knife wire 20 supported by the wire support portion 45 of the handle body portion 42 to the proximal end side. Due to this tension, the distal end portion of the tube 10 fixed to the distal end portion of the knife wire 20 is bent in an arc shape. It is noted that when the handle body portion 42 is returned to the distal end side, the tension of the knife wire 20 is relaxed, and the distal end portion of the tube 10 returns to a substantially linear shape.

### (Procedure Using Medical Device 300)

Next, a procedure for incising a papilla using the medical device 300 in endoscopic sphincterotomy will be described. First, an operator inserts the endoscope 200 to the duodenum, and delivers a guide wire (combination device) inserted into the endoscope 200 from an opening of the papilla to a common bile duct. Next, the operator delivers the incision instrument 100 to the opening of the papilla along the guide wire. Specifically, after inserting a proximal end of the guide wire into a distal end opening of the device lumen 16L of the incision instrument 100, the incision instrument 100 is inserted into the treatment instrument lumen 222 from the insertion port 214 of the endoscope 200, and the incision instrument 100 is advanced to the distal end side until a distal end of the incision instrument 100 projects from the opening 232 of the distal end portion 230 of the endoscope 200 and reaches the opening of the papilla. At this time, the operator positions the handle body portion 42 on the distal end side (FIG. 3). As a result, the distal end portion of the tube 10 becomes linear, and it is possible to prevent the distal end portion of the tube 10 from being caught on the papilla or the common bile duct during delivery of the incision instrument 100.

Next, the operator rotates the distal end portion of the tube 10 by rotating the handle body portion 42 of the handle portion 40 relative to the tube 10 (FIG. 6, arrow AR2), and sets the direction of the exposed part 20E of the knife wire 20 to a direction appropriate for incising the papilla.

Thereafter, the operator applies tension to the knife wire 20 by sliding the handle body portion 42 to the proximal end side along the outer peripheral surface of the shaft portion 41 (FIG. 7, arrow AR1). As a result, the distal end portion of the tube 10 is bent in an arc shape, and the exposed part 20E of the knife wire 20 is brought into a state of being pressed against an incision site. In this state, the operator applies a high-frequency current to the knife wire 20 with a high-frequency power source. As a result, a predetermined range of the papilla is incised into a predetermined shape by the exposed part 20E of the knife wire 20.

As described above, the insertion portion 220 of the endoscope 200 has the curved part 224. The operator bends the curved part 224, for example, according to a shape of an intracorporeal lumen (FIG. 2). When the curved part 224 of the endoscope 200 is bent, the tube 10 of the incision instrument 100 inserted into the treatment instrument lumen 222 of the endoscope 200 is also bent. The rotation operation for changing the direction of the exposed part 20E of the knife wire 20 described above is also performed in a state where the tube 10 of the incision instrument 100 is bent.

### (Detailed Configuration of Knife Wire 20)

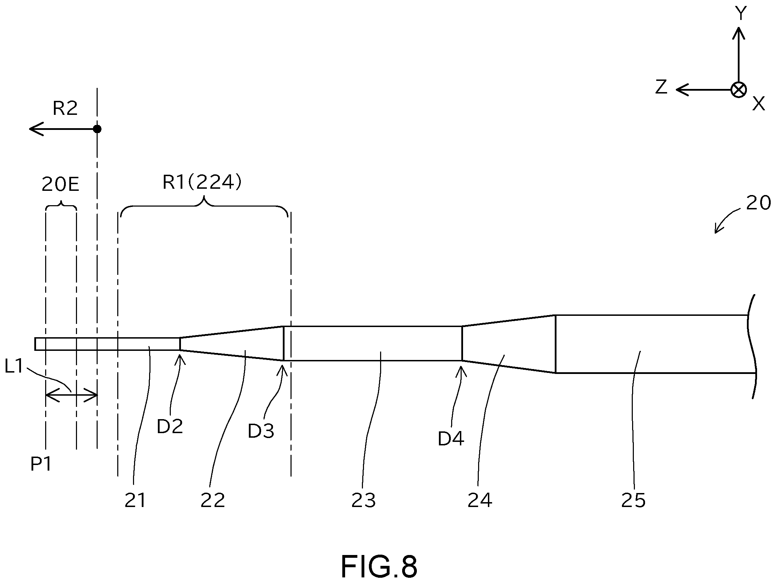

FIG. 8 is an explanatory diagram illustrating a detailed configuration of the knife wire 20 in the first embodiment. FIG. 8 illustrates an external configuration of a distal end portion of the knife wire 20.

The knife wire 20 of the present embodiment has a first wire part 21, a second wire part 22, a third wire part 23, a fourth wire part 24, and a fifth wire part 25, which are arranged in this order from a distal end side toward a proximal end side. The first wire part 21 is a portion including the exposed part 20E. A length of the first wire part 21 is, for example, about 60 mm to 80 mm, a length of the second wire part 22 is, for example, about 10 mm to 30 mm, a length of the third wire part 23 is, for example, about 25 mm to 45 mm, and a length of the fourth wire part 24 is, for example, about 20 mm to 40 mm.

The first wire part 21, the third wire part 23, and the fifth wire part 25 are each cylindrical portions having a substantially constant outer diameter. An outer diameter of the first wire part 21 is, for example, about 0.15 mm to 0.26 mm. An outer diameter of the third wire part 23 is larger than the outer diameter of the first wire part 21, and is, for example, about 0.26 mm to 0.40 mm. An outer diameter of the fifth wire part 25 is larger than the outer diameter of the third wire part 23, and is, for example, about 0.40 mm to 0.80 mm.

The second wire part 22 and the fourth wire part 24 are tapered portions that widen from the distal end side toward the proximal end side. An outer diameter of a distal end of the second wire part 22 is substantially the same as an outer diameter of a proximal end of the first wire part 21, and an outer diameter of a proximal end of the second wire part 22 is substantially the same as an outer diameter of a distal end of the third wire part 23. An outer diameter of a distal end of the fourth wire part 24 is substantially the same as an outer diameter of a proximal end of the third wire part 23, and an outer diameter of a proximal end of the fourth wire part 24 is substantially the same as an outer diameter of a distal end of the fifth wire part 25.

Since each portion of the knife wire 20 has such a configuration, the knife wire 20 has a configuration in which rigidity gradually increases from the distal end side toward the proximal end side. For example, rigidity of the second wire part 22 is higher than rigidity of the first wire part 21, rigidity of the third wire part 23 is higher than the rigidity of the second wire part 22, rigidity of the fourth wire part 24 is higher than the rigidity of the third wire part 23, and rigidity of the fifth wire part 25 is higher than the rigidity of the fourth wire part 24.

FIG. 8 illustrates a positional relationship between the knife wire 20 and the endoscope 200 in a state where the incision instrument 100 is inserted into the treatment instrument lumen 222 of the endoscope 200. More specifically, FIG. 8 illustrates a positional relationship between the knife wire 20 and the endoscope 200 in a state (hereinafter, referred to as a "specific state") in which the incision instrument 100 is inserted into the treatment instrument lumen 222 and a portion (hereinafter, referred to as a "projecting portion R2") from a position P1 of a distal end of the exposed part 20E of the knife wire 20 to a proximal end side by a predetermined distance L1 is projected from the opening 232 of the insertion portion 220 of the endoscope 200. The specific state is a standard state when performing a procedure by projecting a distal end portion of the incision instrument 100 inserted into the treatment instrument lumen 222 of the endoscope 200 from the opening 232 of the distal end portion 230 of the endoscope 200. The predetermined distance L1 is, for example, 44 mm.

In the specific state, a portion (hereinafter, referred to as a "curved-part inner portion R1") of the knife wire 20 disposed in the curved part 224 of the insertion portion 220 of the endoscope 200 is illustrated in FIG. 8. As illustrated in FIG. 8, in the specific state, the curved-part inner portion R1 disposed in the curved part 224 of the endoscope 200 is composed of a proximal end portion of the first wire part 21, the entire second wire part 22, and a distal end portion of the third wire part 23. Therefore, in the specific state, a distal end D2 of the second wire part 22 is located on a more distal end side than a proximal end of the curved-part inner portion R1 (curved part 224). In the present embodiment, in the specific state, the distal end D2 of the second wire part 22 is located on a more distal end side than a central position in a longitudinal direction of the curved-part inner portion R1 (curved part 224). In the specific state, the distal end D2 of the second wire part 22 is located on a more proximal end side than a distal end of the curved-part inner portion R1 (curved part 224).

In the specific state, a distal end D3 of the third wire part 23 is located on a more distal end side than the proximal end of the curved-part inner portion R1 (curved part 224). In the present embodiment, in the specific state, the distal end D3 of the third wire part 23 is located on a more proximal end side than the distal end of the curved-part inner portion R1 (curved part 224). A distal end D4 of the fourth wire part 24 is located on a more proximal end side than the proximal end of the curved-part inner portion R1 (curved part 224).

### (Effects of Present Embodiment)

As described above, the medical device 300 of the present embodiment includes the endoscope 200 having the insertion portion 220 to be inserted into a subject, and the incision instrument 100 as an endoscopic treatment instrument. The insertion portion 220 of the endoscope 200 has the treatment instrument lumen 222 that opens at the distal end portion 230 of the insertion portion 220 and into which the incision instrument 100 is inserted, and the curved part 224 that is located on a more proximal end side than the distal end portion 230 and is bent by a bending operation. The incision instrument 100 has the tube 10 having the knife wire lumen 17L, the knife wire 20 housed in the knife wire lumen 17L, and the handle body portion 42 as a support member that rotatably supports a proximal end portion of the knife wire 20. The knife wire 20 includes the first wire part 21 including the exposed part 20E that can be exposed to an outer peripheral side of the tube 10, and the second wire part 22 that is located on a proximal end side of the first wire part 21 and has higher rigidity than the exposed part 20E of the first wire part 21. In the specific state in which the incision instrument 100 is inserted into the treatment instrument lumen 222 of the endoscope 200 and the projecting portion R2, which is a portion from the position P1 of the distal end of the exposed part 20E of the knife wire 20 to a proximal end side by the predetermined distance L1, is projected from the opening 232 of the insertion portion 220 of the endoscope 200, the distal end D2 of the second wire part 22 is located on a more distal end side than a proximal end of the curved part 224 (curved-part inner portion R1).

Since the medical device 300 of the present embodiment has such a configuration, it is possible to smoothly change the direction of the exposed part 20E of the knife wire 20 to a desired direction by a rotation operation of the handle body portion 42, as will be described below.

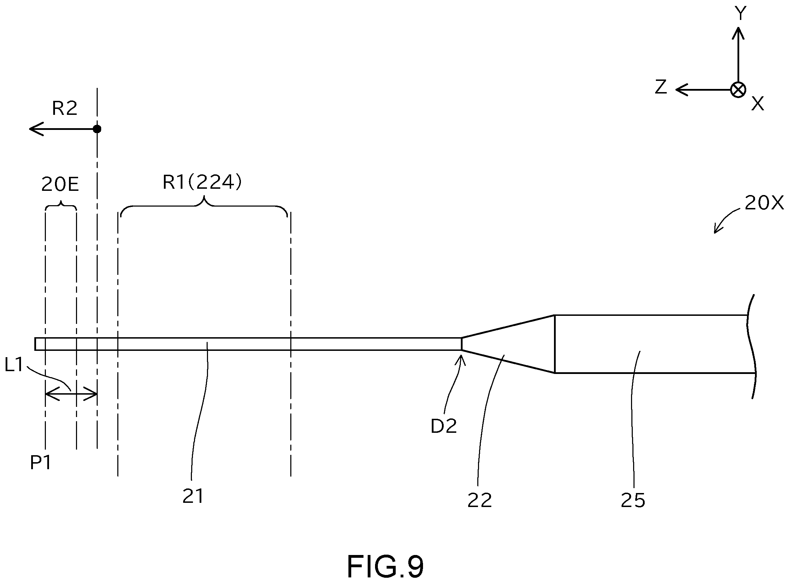

FIG. 9 is an explanatory diagram illustrating a configuration of a knife wire 20X in a comparative example. The knife wire 20X of the comparative example, similarly to the knife wire 20 of the first embodiment, includes a first wire part 21 including an exposed part 20E, and a second wire part 22 that is located on a proximal end side of the first wire part 21 and has higher rigidity than the exposed part 20E of the first wire part 21. In the comparative example, in the specific state, a distal end D2 of the second wire part 22 is located on a more proximal end side than a proximal end of the curved part 224 (curved-part inner portion R1). That is, in the specific state, only the first wire part 21 of the knife wire 20X, which has relatively low rigidity, is located in the curved part 224 of the insertion portion 220 of the endoscope 200. Therefore, in a state where the curved part 224 of the endoscope 200 is bent and the knife wire 20X of the incision instrument 100 housed in the treatment instrument lumen 222 is bent, a rotational torque generated by rotating the handle body portion 42 that supports the proximal end of the knife wire 20X is not favorably transmitted to the distal end side in a portion of the knife wire 20X located in the curved part 224, and it is not possible to smoothly change the direction of the exposed part 20E of the knife wire 20X to a desired direction.

In contrast, in the medical device 300 of the present embodiment, in the specific state, the distal end D2 of the second wire part 22, which has higher rigidity than the first wire part 21 of the knife wire 20, is located on a more distal end side than the proximal end of the curved part 224 of the endoscope 200. That is, in the specific state, a portion of the knife wire 20 having higher rigidity than the first wire part 21 is located in the curved part 224 of the endoscope 200. Therefore, even when the curved part 224 of the endoscope 200 is bent and the knife wire 20 is bent, a rotational torque generated by rotating the handle body portion 42 is favorably transmitted to the distal end side even in a portion of the knife wire 20 located in the curved part, and the direction of the exposed part 20E of the knife wire 20 can be smoothly changed to a desired direction. Therefore, according to the medical device 300 of the present embodiment, it is possible to smoothly change the direction of the exposed part 20E of the knife wire 20 to a desired direction by a rotation operation of the handle body portion 42 while maintaining the inner and outer diameters of the tube 10 and the outer diameter of the knife wire 20.

In the medical device 300 of the present embodiment, in the specific state, the distal end D2 of the second wire part 22 of the knife wire 20 is located on a more distal end side than a central position in a longitudinal direction of the curved part 224 of the endoscope 200. That is, in the specific state, a portion of the knife wire 20 having relatively high rigidity is located over a majority of the curved part 224 of the endoscope 200 in the longitudinal direction. Therefore, even when the curved part 224 of the endoscope 200 is bent and the knife wire 20 is bent, a rotational torque generated by rotating the handle body portion 42 is more favorably transmitted to the distal end side even in a portion of the knife wire 20 located in the curved part, and the direction of the exposed part 20E of the knife wire 20 can be more smoothly changed to a desired direction.

In the medical device 300 of the present embodiment, the first wire part 21 of the knife wire 20 is cylindrical, and the second wire part 22 has a tapered shape that widens from a distal end side toward a proximal end side. Therefore, according to the medical device 300 of the present embodiment, it is possible to provide the second wire part 22 with relatively high rigidity while suppressing a rigidity gap in the knife wire 20.

In the medical device 300 of the present embodiment, the knife wire 20 includes the third wire part 23 and the fourth wire part 24. The third wire part 23 is located on a proximal end side of the second wire part 22, has higher rigidity than a distal end portion of the second wire part 22, and is cylindrical. The fourth wire part 24 is located on a proximal end side of the third wire part 23, has higher rigidity than a distal end portion of the third wire part 23, and has a tapered shape that widens from a distal end side toward a proximal end side. In the specific state, the distal end of the third wire part 23 is located on a more distal end side than a proximal end of the curved part 224, and the distal end of the fourth wire part 24 is located on a more proximal end side than the proximal end of the curved part 224. Therefore, according to the medical device 300 of the present embodiment, the fourth wire part 24 having a relatively large diameter is not disposed in the curved part 224 of the endoscope 200, and a decrease in slidability of the incision instrument 100 in the treatment instrument lumen 222 of the endoscope 200 can be suppressed.

In the medical device 300 of the present embodiment, a minimum value of a diameter of the exposed part 20E of the knife wire 20 is 0.15 mm or more and 0.30 mm or less. Therefore, according to the medical device 300 of the present embodiment, it is possible to achieve both an improvement in rotational torque transmissibility of the knife wire 20 and an improvement in an incision capability of the knife wire 20.

### B. Second Embodiment:

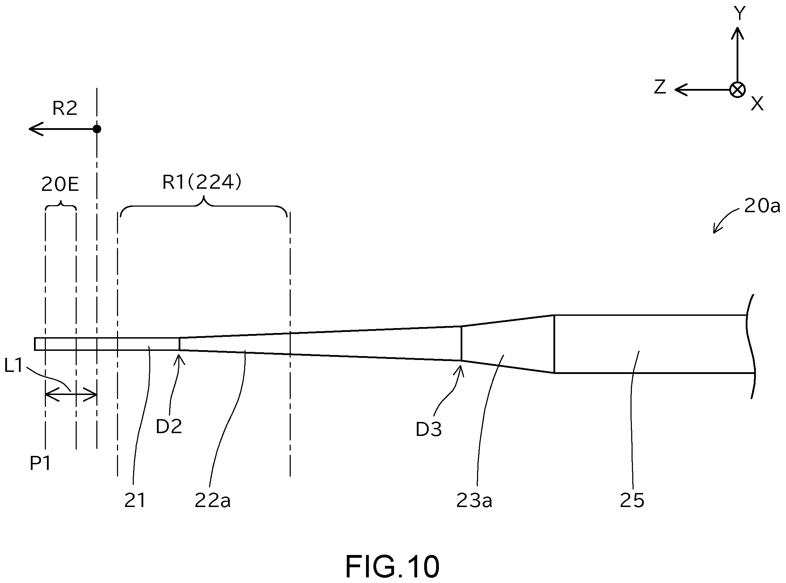

FIG. 10 is an explanatory diagram illustrating a detailed configuration of a knife wire 20a in a second embodiment. Hereinafter, among the configurations of the knife wire 20a of the second embodiment, the same components as those of the knife wire 20 of the first embodiment described above are denoted by the same reference numerals, and description thereof will be omitted as appropriate.

The knife wire 20a of the second embodiment has a first wire part 21, a second wire part 22a, a third wire part 23a, and a fifth wire part 25, which are arranged in this order from a distal end side toward a proximal end side. The first wire part 21 is a portion including the exposed part 20E. A length of the first wire part 21 is, for example, about 60 mm to 80 mm, a length of the second wire part 22a is, for example, about 45 mm to 65 mm, and a length of the third wire part 23a is, for example, about 20 mm to 40 mm.

The first wire part 21 and the fifth wire part 25 are cylindrical portions having a substantially constant outer diameter. On the other hand, the second wire part 22a and the third wire part 23a are tapered portions that widen from a distal end side toward a proximal end side. A degree of widening (inclination) from the distal end side toward the proximal end side in the third wire part 23a is larger than a degree of widening (inclination) in the second wire part 22a. An outer diameter of a distal end of the second wire part 22a is substantially the same as an outer diameter of a proximal end of the first wire part 21, and an outer diameter of a proximal end of the second wire part 22a is substantially the same as an outer diameter of a distal end of the third wire part 23a. An outer diameter of a proximal end of the third wire part 23a is substantially the same as an outer diameter of a distal end of the fifth wire part 25.

Since each portion of the knife wire 20a has such a configuration, the knife wire 20a has a configuration in which rigidity gradually increases from the distal end side toward the proximal end side. For example, rigidity of the second wire part 22a is higher than rigidity of the first wire part 21, rigidity of the third wire part 23a is higher than the rigidity of the second wire part 22a, and rigidity of the fifth wire part 25 is higher than the rigidity of the third wire part 23a.

In the specific state described above, the curved-part inner portion R1 disposed in the curved part 224 of the endoscope 200 is composed of a proximal end portion of the first wire part 21 and a distal end portion of the second wire part 22a. Therefore, in the specific state, a distal end D2 of the second wire part 22a is located on a more distal end side than a proximal end of the curved-part inner portion R1 (curved part 224). In the present embodiment, in the specific state, the distal end D2 of the second wire part 22a is located on a more distal end side than a central position in a longitudinal direction of the curved-part inner portion R1 (curved part 224).

In the specific state, a distal end D3 of the third wire part 23a is located on a more proximal end side than the proximal end of the curved-part inner portion R1 (curved part 224).

Also in the second embodiment, similarly to the first embodiment, since the distal end D2 of the second wire part 22a is located on a more distal end side than the proximal end of the curved part 224 in the specific state, even when the curved part 224 of the endoscope 200 is bent and the knife wire 20a is bent, a rotational torque generated by rotating the handle body portion 42 is favorably transmitted to the distal end side even in a portion of the knife wire 20a located in the curved part 224, and the direction of the exposed part 20E of the knife wire 20a can be smoothly changed to a desired direction.

In the second embodiment, the knife wire 20a includes the third wire part 23a. The third wire part 23a is located on a proximal end side of the second wire part 22a, has higher rigidity than a distal end portion of the second wire part 22a, and has a tapered shape that widens from a distal end side toward a proximal end side. In the specific state, the distal end D3 of the third wire part 23a is located on a more proximal end side than a proximal end of the curved part 224. Therefore, according to the second embodiment, the third wire part 23a having a relatively large diameter is not disposed in the curved part 224 of the endoscope 200, and a decrease in slidability of the incision instrument 100 in the treatment instrument lumen 222 of the endoscope 200 can be suppressed.

### C. Third Embodiment:

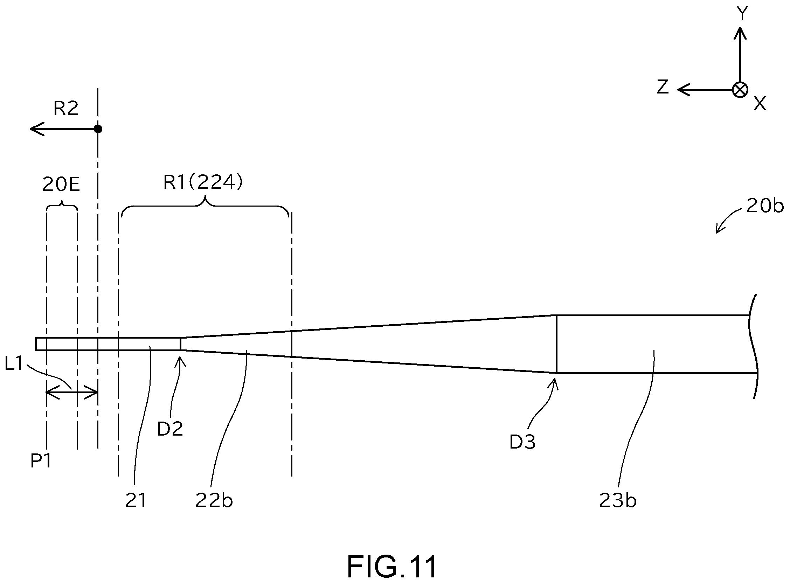

FIG. 11 is an explanatory diagram illustrating a detailed configuration of a knife wire 20b in a third embodiment. Hereinafter, among the configurations of the knife wire 20b of the third embodiment, the same components as those of the knife wire 20 of the first embodiment described above are denoted by the same reference numerals, and description thereof will be omitted as appropriate.

The knife wire 20b of the third embodiment has a first wire part 21, a second wire part 22b, and a third wire part 23b, which are arranged in this order from a distal end side toward a proximal end side. The first wire part 21 is a portion including the exposed part 20E. A length of the first wire part 21 is, for example, about 60 mm to 80 mm, and a length of the second wire part 22b is, for example, about 75 mm to 95 mm.

The first wire part 21 and the third wire part 23b are cylindrical portions having a substantially constant outer diameter. On the other hand, the second wire part 22b is a tapered portion that widens from a distal end side toward a proximal end side. An outer diameter of a distal end of the second wire part 22b is substantially the same as an outer diameter of a proximal end of the first wire part 21, and an outer diameter of a proximal end of the second wire part 22b is substantially the same as an outer diameter of a distal end of the third wire part 23b.

Since each portion of the knife wire 20b has such a configuration, the knife wire 20b has a configuration in which rigidity gradually increases from the distal end side toward the proximal end side. For example, rigidity of the second wire part 22b is higher than rigidity of the first wire part 21, and rigidity of the third wire part 23b is higher than the rigidity of the second wire part 22b.

In the specific state described above, the curved-part inner portion R1 disposed in the curved part 224 of the endoscope 200 is composed of a proximal end portion of the first wire part 21 and a distal end portion of the second wire part 22b. Therefore, in the specific state, a distal end D2 of the second wire part 22b is located on a more distal end side than a proximal end of the curved-part inner portion R1 (curved part 224). In the present embodiment, in the specific state, the distal end D2 of the second wire part 22b is located on a more distal end side than a central position in a longitudinal direction of the curved-part inner portion R1 (curved part 224).

In the specific state, a distal end D3 of the third wire part 23b is located on a more proximal end side than the proximal end of the curved-part inner portion R1 (curved part 224).

Also in the third embodiment, similarly to the first embodiment, since the distal end D2 of the second wire part 22b is located on a more distal end side than the proximal end of the curved part 224 in the specific state, even when the curved part 224 of the endoscope 200 is bent and the knife wire 20b is bent, a rotational torque generated by rotating the handle body portion 42 is favorably transmitted to the distal end side even in a portion of the knife wire 20b located in the curved part 224, and the direction of the exposed part 20E of the knife wire 20b can be smoothly changed to a desired direction.

In the third embodiment, the knife wire 20b includes the third wire part 23b. The third wire part 23b is located on a proximal end side of the second wire part 22b, has higher rigidity than a distal end portion of the second wire part 22b, and is cylindrical. In the specific state, the distal end D3 of the third wire part 23b is located on a more proximal end side than a proximal end of the curved part 224. Therefore, according to the third embodiment, the third wire part 23b having a relatively large diameter is not disposed in the curved part 224 of the endoscope 200, and a decrease in slidability of the incision instrument 100 in the treatment instrument lumen 222 of the endoscope 200 can be suppressed.

### D. Example:

Two incision instruments 100 were created using the knife wire 20 of the first embodiment described above and the knife wire 20X of the comparative example. A test was conducted for each of the two incision instruments 100 to determine whether the direction of the exposed part 20E of the knife wire could be smoothly changed to a desired direction. Specifically, the incision instrument 100 was inserted into the treatment instrument lumen 222 of the endoscope 200, and a distal end portion of the incision instrument 100 was projected from the opening 232 of the distal end portion 230 of the endoscope 200 so as to be in the specific state described above. At this time, a predetermined distance L1 (see FIG. 8) that defines the projecting portion R2 of the knife wire 20 was set to 44 mm. The curved part 224 of the endoscope 200 was bent so that a bending angle θ1 (FIG. 2) became 60 degrees. In this state, the handle body portion 42 of the incision instrument 100 was rotated to change the direction of the exposed part 20E of the knife wire 20. A rotation angle of the handle body portion 42 required to change the direction of the exposed part 20E of the knife wire 20 by 180 degrees was examined.

In the incision instrument 100 created using the knife wire 20X of the comparative example, it was necessary to rotate the handle body portion 42 about 6 times in order to change the direction of the exposed part 20E of the knife wire 20X by 180 degrees. On the other hand, in the incision instrument 100 created using the knife wire 20 of the first embodiment, it was necessary to rotate the handle body portion 42 about 5 times in order to change the direction of the exposed part 20E of the knife wire 20 by 180 degrees.

This test confirmed that by adopting a configuration in which the distal end D2 of the second wire part 22 is located on a more distal end side than the proximal end of the curved part 224 in the specific state, even when the curved part 224 of the endoscope 200 is bent and the knife wire 20 is bent, a rotational torque generated by rotating the handle body portion 42 is favorably transmitted to the distal end side, and the direction of the exposed part 20E of the knife wire 20 can be smoothly changed to a desired direction.

### E. Modified Examples:

The technology disclosed in the present specification is not limited to the above-described embodiments, and can be modified into various forms without departing from the gist thereof, and for example, the following modifications are also possible.

The configuration of the medical device 300 in the above-described embodiments is merely an example, and can be variously modified. For example, in the above-described embodiments, a difference in rigidity of each portion of the knife wire 20 is realized by a difference in outer diameter, but the difference in rigidity of each portion may be realized by other means. For example, the difference in rigidity of each portion of the knife wire 20 may be realized by a difference in forming material, or may be realized by a difference in mechanical structure such as a wire and a rope.

In the above-described embodiments, in the specific state, the distal end D2 of the second wire part 22 of the knife wire 20 is located on a more proximal end side than a distal end of the curved part 224, but the distal end D2 of the second wire part 22 may be located on a more distal end side than the distal end of the curved part 224.

In the above-described embodiments, the opening 232 of the treatment instrument lumen 222 is formed on an outer peripheral surface of the distal end portion 230 of the insertion portion 220, but the opening 232 may be formed on a distal end surface of the distal end portion 230. The treatment instrument lumen 222 opening at the distal end portion 230 of the insertion portion 220 includes a form in which the treatment instrument lumen 222 opens at the outer peripheral surface of the distal end portion 230 of the insertion portion 220, and also a form in which the treatment instrument lumen 222 opens at an other surface (for example, a distal end surface) of the distal end portion 230.

In the above-described embodiments, a cross section of each portion of the knife wire 20 is substantially circular, but the cross section of each portion of the knife wire 20 may have any other shape such as a substantially elliptical shape or a polygonal shape.

In the above-described embodiments, the knife wire lumen 17L, the device lumen 16L, and the liquid feeding lumen 18L are formed in the tube 10, but the device lumen 16L and/or the liquid feeding lumen 18L may not be formed.

In the above-described embodiments, the exposed part 20E of the knife wire 20 is always exposed outside of the tube 10, but the exposed part 20E of the knife wire 20 may be exposed outside of the tube 10 at least when the tube 10 is in a predetermined posture (for example, a greatly bent posture), and does not need to be always exposed outside.

## Claims

1. A medical device (300) comprising:
an endoscope (200) having an insertion portion (220) to be inserted into a subject; and
an endoscopic treatment instrument (100),
wherein the insertion portion (220) of the endoscope (200) includes:
a treatment instrument lumen (222) that opens at a distal end portion (230) of the insertion portion (220) and into which the endoscopic treatment instrument (100) is inserted; and
a curved part (224) that is located on a more proximal end side than the distal end portion (230) and is bent by a bending operation,
wherein the endoscopic treatment instrument (100) includes:
a tube (10) having a knife wire lumen (17L);
a knife wire (20, 20a, 20b) housed in the knife wire lumen (17L); and
a support member (42) that rotatably supports a proximal end portion (29) of the knife wire (20, 20a, 20b),
wherein the knife wire (20, 20a, 20b) includes:
a first wire part (21) including an exposed part (20E) that can be exposed to an outer peripheral side of the tube (10); and
a second wire part (22, 22a, 22b) that is located on a more proximal end side than the first wire part (21) and has higher rigidity than the exposed part (20E) of the first wire part (21), and
wherein in a specific state in which the endoscopic treatment instrument (100) is inserted into the treatment instrument lumen (222) and a portion from a position (P1) of a distal end of the exposed part (20E) of the knife wire (20, 20a, 20b) exposed from the tube (10) to a proximal end side by a predetermined distance (L1) is projected from an opening (232) of the insertion portion (220), a distal end (D2) of the second wire part (22, 22a, 22b) is located on a more distal end side than a proximal end of the curved part (224).

2. The medical device (300) according to claim 1,
wherein in the specific state, the distal end (D2) of the second wire part (22, 22a, 22b) is located on a more distal end side than a central position in a longitudinal direction of the curved part (224).

3. The medical device (300) according to claim 1 or 2,
wherein the first wire part (21) is cylindrical, and the second wire part (22, 22a, 22b) has a tapered shape that widens from a distal end side toward a proximal end side.

4. The medical device (300) according to claim 3,
wherein the knife wire (20a) includes a third wire part (23a) that is located on a more proximal end side than the second wire part (22a), has higher rigidity than a distal end portion of the second wire part (22a), and has a tapered shape that widens from a distal end side toward a proximal end side, and
wherein in the specific state, a distal end (D3) of the third wire part (23a) is located on a more proximal end side than a proximal end of the curved part (224).

5. The medical device (300) according to claim 3,
wherein the knife wire (20b) includes a third wire part (23b) that is located on a more proximal end side than the second wire part (22b), has higher rigidity than a distal end portion of the second wire part (22b), and is cylindrical, and
wherein in the specific state, a distal end (D3) of the third wire part (23b) is located on a more proximal end side than a proximal end of the curved part (224).

6. The medical device (300) according to claim 3,
wherein the knife wire (20) includes:
a third wire part (23) that is located on a more proximal end side than the second wire part (22), has higher rigidity than a distal end portion of the second wire part (22), and is cylindrical; and
a fourth wire part (24) that is located on a more proximal end side than the third wire part (23), has higher rigidity than a distal end portion of the third wire part (23), and has a tapered shape that widens from a distal end side toward a proximal end side, and
wherein in the specific state, a distal end (D3) of the third wire part (23) is located on a more distal end side than a proximal end of the curved part (224), and a distal end (D4) of the fourth wire part (24) is located on a more proximal end side than the proximal end of the curved part (224).

7. The medical device (300) according to any one of claims 1 to 6,
wherein a minimum value of a diameter of the exposed part (20E) of the knife wire (20, 20a, 20b) is 0.15 mm or more and 0.30 mm or less.

8. An endoscopic treatment instrument (100) used in combination with an endoscope (200) having an insertion portion (220) to be inserted into a subject,
wherein the insertion portion (220) of the endoscope (200) includes:
a treatment instrument lumen (222) that opens at a distal end portion (230) of the insertion portion (220) and into which the endoscopic treatment instrument (100) is inserted; and
a curved part (224) that is located on a more proximal end side than the distal end portion (230) and is bent by a bending operation,
wherein the endoscopic treatment instrument (100) includes:
a tube (10) having a knife wire lumen (17L);
a knife wire (20, 20a, 20b) housed in the knife wire lumen (17L); and
a support member (42) that rotatably supports a proximal end portion (29) of the knife wire (20, 20a, 20b),
wherein the knife wire (20, 20a, 20b) includes:
a first wire part (21) including an exposed part (20E) that can be exposed to an outer peripheral side of the tube (10); and
a second wire part (22, 22a, 22b) that is located on a more proximal end side than the first wire part (21) and has higher rigidity than the exposed part (20E) of the first wire part (21), and
wherein in a specific state in which the endoscopic treatment instrument (100) is inserted into the treatment instrument lumen (222) and a portion from a position (P1) of a distal end of the exposed part (20E) of the knife wire (20, 20a, 20b) exposed from the tube (10) to a proximal end side by a predetermined distance (L1) is projected from an opening (232) of the insertion portion (220), a distal end (D2) of the second wire part (22, 22a, 22b) is located on a more distal end side than a proximal end of the curved part (224).

9. The endoscopic treatment instrument (100) according to claim 8,
wherein in the specific state, the distal end (D2) of the second wire part (22, 22a, 22b) is located on a more distal end side than a central position in a longitudinal direction of the curved part (224).

10. The endoscopic treatment instrument (100) according to claim 8 or 9,
wherein the first wire part (21) is cylindrical, and the second wire part (22, 22a, 22b) has a tapered shape that widens from a distal end side toward a proximal end side.

11. The endoscopic treatment instrument (100) according to claim 10,
wherein the knife wire (20a) includes a third wire part (23a) that is located on a more proximal end side than the second wire part (22a), has higher rigidity than a distal end portion of the second wire part (22a), and has a tapered shape that widens from a distal end side toward a proximal end side, and
wherein in the specific state, a distal end (D3) of the third wire part (23a) is located on a more proximal end side than a proximal end of the curved part (224).

12. The endoscopic treatment instrument (100) according to claim 10,
wherein the knife wire (20b) includes a third wire part (23b) that is located on a more proximal end side than the second wire part (22b), has higher rigidity than a distal end portion of the second wire part (22b), and is cylindrical, and
wherein in the specific state, a distal end (D3) of the third wire part (23b) is located on a more proximal end side than a proximal end of the curved part (224).

13. The endoscopic treatment instrument (100) according to claim 10,
wherein the knife wire (20) includes:
a third wire part (23) that is located on a more proximal end side than the second wire part (22), has higher rigidity than a distal end portion of the second wire part (22), and is cylindrical; and
a fourth wire part (24) that is located on a more proximal end side than the third wire part (23), has higher rigidity than a distal end portion of the third wire part (23), and has a tapered shape that widens from a distal end side toward a proximal end side, and
wherein in the specific state, a distal end (D3) of the third wire part (23) is located on a more distal end side than a proximal end of the curved part (224), and a distal end (D4) of the fourth wire part (24) is located on a more proximal end side than the proximal end of the curved part (224).

14. The endoscopic treatment instrument (100) according to any one of claims 8 to 13,
wherein a minimum value of a diameter of the exposed part (20E) of the knife wire (20, 20a, 20b) is 0.15 mm or more and 0.30 mm or less.
